# EUROPEAN PATENT APPLICATION

(11) **EP 2 100 558 A1**
(43) Date of publication of application: **16.09.2009**
(21) Application number: 08004420.9
(22) Date of filing: 10.03.2008
(51) Int. Cl.: A61B 5/103, A61B 8/08

(54) **Pressing mechanism and ultrasound diagnostic apparatus**

(71) Applicant: Aloka Co., Ltd., Mitaka-shi Tokyo 181-8622 (JP)
(72) Inventor: Otsuka, Toshiki, Mitaki-shi Tokyo, 181-8622 (JP)
(74) Representative: Heim, Hans-Karl

(57) **Abstract**

An ultrasound diagnostic apparatus according to the present invention includes a pressing mechanism capable of appropriately applying a pressing force to a predetermined position on a fractured bone. The pressing mechanism includes an annular belt detachably hung around a leg in which a shinbone (i.e., a target bone) is present, a deadweight connected to a lower end of the annular belt and having a weight value corresponding to a desired pressing force, and a mobile base positioned under the deadweight and movable in an up-and-down direction. If the mobile base moves downward, the deadweight separates from the mobile base and is brought into a free state where a pressing force acts on the target bone. If the mobile base moves upward, the deadweight is supported by the mobile base and the target bone is released from the pressing force.

## Description

### BACKGROUND OF THE INVENTION

### 1. Field of the Invention

The present invention relates to a pressing mechanism that applies a pressing force to a target bone in the diagnosis of mechanical properties, and also relates to an ultrasound diagnostic apparatus including the pressing mechanism.

### 2. Description of the Related Art

Easy quantitative measurement of mechanical properties is desired for diagnosing bone metabolic diseases such as osteoporosis, judging fracture risk, and quantitatively diagnosing bone union after treatment of bone fracture. The evaluation of mechanical properties of a bone (including strength and elasticity) greatly depends on X-ray photography, but quantitatively diagnosing bone strength by means of X-ray photography is very difficult. X-ray irradiation may also have adverse effects on the human body.

On the other hand, to simply evaluate mechanical properties (e.g., bone strength) of a bone, an ultrasound diagnostic apparatus discussed in Japanese Patent Laid-Open Publication No. 2004-298205 measures the shape of a target bone which deforms when a stress is applied, and diagnoses mechanical properties of the bone based on a measurement result.

A conventional diagnostic apparatus includes a dedicated pressing mechanism that can control a load value applied to a target bone and a position where the load is added, in the diagnosis of mechanical properties. For example, a conventional pressing mechanism includes a rigid pressing member which can be brought into contact with a surface of a human body which includes a bone to be pressed. The pressing mechanism is configured to control a load value applied to the bone by adjusting an advancing amount of the pressing member relative to the body.

A user can evaluate bone union of a fractured bone if mechanical properties of the bone are obtainable through the above-described diagnosis. In other words, the diagnosis of mechanical properties can be preferably executed for a fractured bone. In general, a plaster or an external fixation device can tightly hold a fractured bone. The external fixation device includes a plurality of screws and pins which are partly inserted into a human body to fix the bone in position. The external fixation device is fixed to the body in a stationary manner unless the fractured bone is completely cured.

In a state where an external fixation device is attached around a target body, a user cannot smoothly perform the ultrasonic mechanical property diagnosis on a fractured bone. Namely, if an external fixation device is attached around a target body, the above-described pressing mechanism of the diagnostic apparatus interferes with metal fittings of the external fixation device in the vicinity of the body. As a result, a user cannot apply an appropriate load to the fractured bone. Furthermore, regardless of the presence of an external fixation device, a conventional pressing mechanism is unable to apply an appropriate pressing force to a target bone if the type of diagnosis object is inappropriate, or if another method or device for curing a fractured bone is employed.

### SUMMARY OF THE INVENTION

The present invention is directed to a pressing mechanism capable of accurately pressing a predetermined pressing position, and an ultrasound diagnostic apparatus capable of appropriately performing diagnosis of mechanical properties.

According to an aspect of the present invention, a pressing mechanism applies a pressing force to a target bone when a diagnosis of mechanical properties is performed for the target bone. The pressing mechanism includes an annular belt detachably hung around a diagnosis object in which the target bone is present, a deadweight connected to a lower end of the annular belt and having a weight value corresponding to a desired pressing force, and a mobile base positioned under the deadweight and movable in an up-and-down direction. If the mobile base moves downward, the deadweight separates from the mobile base and is brought into a free state where a pressing force acts on the target bone. If the mobile base moves upward, the deadweight is supported by the mobile base and the target bone is released from the pressing force.

In a preferred embodiment, the annular belt is entrained around a plurality of rollers which are spaced from the diagnosis object. In this case, the rollers include a group of fixed rollers which are symmetrically disposed with respect to a pressing position in the right-and-left direction, and are in a fixed relationship relative to the annular belt, and a group of movable rollers which can slide along an inner surface of the annular belt and automatically move to a position where the gravity acting on the annular belt is balanced in the right-and-left direction.

In a preferred embodiment, the mobile base supports the deadweight via a plurality of balls that permits horizontal movement of the deadweight relative to the mobile base. In a preferred embodiment, an urging member is disposed between the mobile base and the deadweight to urge the deadweight in the upward direction.

According to another aspect of the present invention, an ultrasound diagnostic apparatus is configured to transmit/receive ultrasonic waves to/from a target bone in a state where a pressing force is applied to the target bone, and to obtain deformation properties of the target bone subjected to the pressing force. The ultrasound diagnostic apparatus includes a pressing mechanism configured to press a predetermined position on a target bone, an ultrasonic probe configured to transmit and receive ultrasonic wave to and from the target bone, and a calculation unit configured to calculate a change in the target bone before and after the target bone is pressed by the pressing mechanism based on an echo signal obtained via the ultrasonic probe that performs transmission/reception of ultrasonic wave. The pressing mechanism includes an annular belt detachably hung around a diagnosis object in which the target bone is present, a deadweight connected to a lower end of the annular belt and having a weight value corresponding to a desired pressing force, and a mobile base positioned under the deadweight and movable in an up-and-down direction. If the mobile base moves downward, the deadweight separates from the mobile base and is brought into a free state where a pressing force acts on the target bone. If the mobile base moves upward, the deadweight is supported by the mobile base and the target bone is released from the pressing force.

According to the present invention, the gravity of a deadweight hung around a diagnosis object can be applied as a pressing force via an annular belt to a target bone. The annular belt is sufficiently compact in size and can be positioned closely to a pressing position. As a result, the main body of the pressing mechanism does not interfere with an external fixation device or another medical instrument. Thus, the pressing mechanism can appropriately apply a pressing force to a predetermined pressing position.

### BRIEF DESCRIPTION OF THE DRAWINGS

Exemplary embodiments of the present invention will be described in detail based on the following figures, wherein:
Fig. 1 is a block diagram illustrating an ultrasound diagnostic apparatus according to an embodiment of the present invention;
Fig. 2 illustrates example tracking processing for obtaining echo signals from a bone surface;
Fig. 3 illustrates a perspective view of an example external fixation device;
Fig. 4 illustrates a perspective view of a pressing mechanism;
Fig. 5 illustrates a cross-sectional view of the pressing mechanism;
Fig. 6 illustrates a cross-sectional view taken along a line A-A of Fig. 5;
Fig. 7A illustrates a partly enlarged view of another pressing mechanism; and
Fig. 7B illustrates a partly enlarged view of another pressing mechanism.

### DESCRIPTION OF PREFERRED EMBODIMENTS

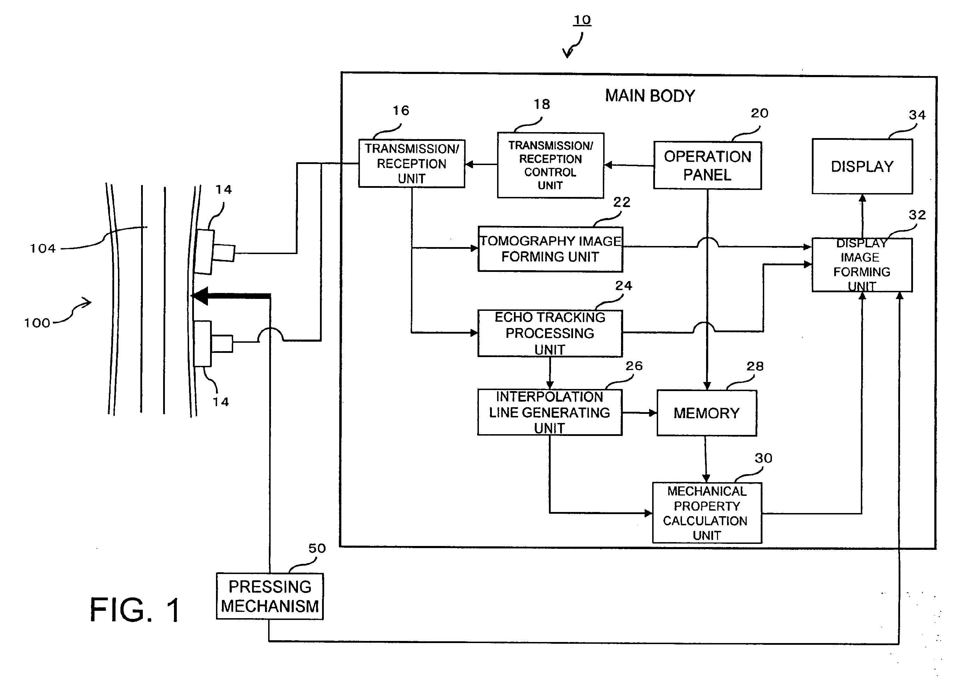

An embodiment of the present invention is described below with reference to the drawings. Fig. 1 is a block diagram illustrating an ultrasound diagnostic apparatus according to an embodiment of the present invention. An ultrasound diagnostic apparatus 10 is configured to diagnose mechanical properties of a bone (e.g., diagnosis object). For example, the diagnosis object is a shinbone 104 in a human leg 100.

The ultrasound diagnostic apparatus 10 according to the present embodiment includes two probes 14 which are brought into contact with a body surface of a measurement target. The probe 14 is, for example, an ultrasonic probe capable of emitting ultrasonic beams toward the shinbone 104 (target bone) inside the body of the measurement target.

A transmission/reception unit 16 controls each probe 14 that performs electronic scanning with ultrasonic beams on a tomography surface. For example, if the probe 14 is a linear probe, the probe 14 successively emits a total of 120 ultrasonic beams for electronic scanning. The transmission/reception unit 16 obtains an echo signal from each ultrasonic beam. A transmission/reception control unit 18 controls the transmission/reception unit 16 according to a user's instruction input via an operation panel 20.

The transmission/reception unit 16 outputs a plurality of echo signals (i.e., the signals having been obtained as described above) to a tomography image forming unit 22. The tomography image forming unit 22 forms a tomography image (B mode image) of a target bone based on the received echo signals. The transmission/reception unit 16 also outputs the obtained echo signals to an echo tracking processing unit 24. The echo tracking processing unit 24 performs echo tracking processing (i.e., extracts bone surface portions from the echo signals and performs tracking). For example, a technique discussed in Japanese Patent Laid-Open Publication No. 2001-309918 can be used for the echo tracking processing. The echo tracking processing uses a plurality of (e.g., three) tracking echo signals. The tracking echo signals can be selected from echo signals used for tomography image formation (e.g., 120 echo signals). Alternatively, three echo signals dedicated to the tracking can be used if available during interruption of the tomography image formation.

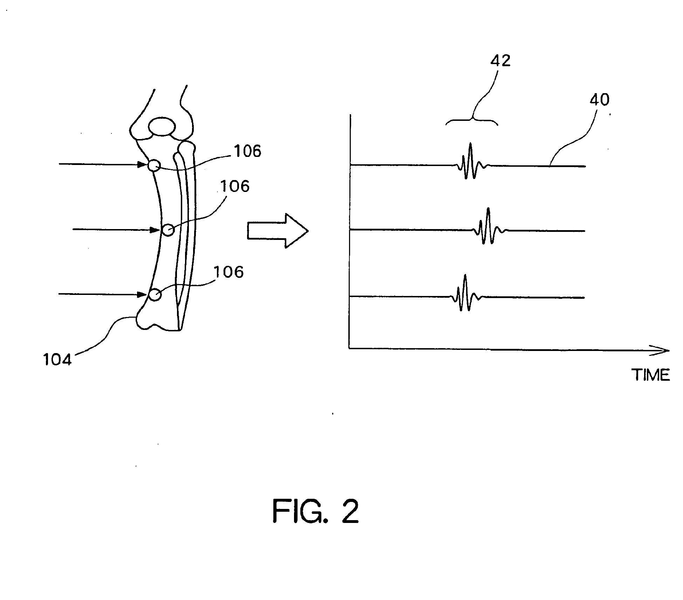

Fig. 2 illustrates example tracking processing for obtaining three echo signals 40 from a surface portion of the bone 104. Three echo signals 40, corresponding to three ultrasonic beams emitted toward the bone 104, have a large value in amplitude (amplitude maximized portion 42) at a portion corresponding to the bone surface. The echo tracking processing unit 24 identifies the position on a bone surface based on the position of the amplitude maximized portion 42 (i.e., acquiring time of a corresponding waveform). Although the example tracking processing illustrated in Fig. 2 uses three echo signals for echo tracking, the number of echo signals used for the measurement can be changed arbitrarily. In the echo tracking processing, a surface point being tracked for each echo signal 40 (i.e., for each ultrasonic beam) can be referred to as tracking point 106.

An interpolation line generation unit 26 (refer to Fig. 1) generates an interpolation line connecting three tracking points 106. Namely, the interpolation line generation unit 26 generates an interpolated curve connecting respective tracking points 106 according to the spline interpolation or the least squares method interpolation. The generated interpolation line is a curve representing the shape of a target bone surface. The interpolation line, which can more accurately represent the surface shape of a target bone, can be obtained if the number of echo signals for the echo tracking processing is increased. The generated interpolation line can be temporarily stored in a memory 28. A mechanical property calculation unit 30 calculates a mechanical property value (e.g., deflection amount) of a target bone with reference to the information (i.e., information relating to the shape of a bone surface) temporarily stored in the memory 28.

A display image forming unit 32 forms an image to be displayed on a display unit 34, as a result of diagnosis. The display image forming unit 32 receives a tomography image formed by the tomography image forming unit 22, a mechanical property value (e.g., deflection amount) calculated by the mechanical property calculation unit 30, and a load value applied to the target bone from the pressing mechanism 50. The display image forming unit 32 forms a display image of a graph representing the received mechanical property value and/or the load value, and outputs the display image to the display unit 34. For example, the display image forming unit 32 directly outputs the display image to the display unit 34. Alternatively, the display image forming unit 32 can combine the display image with the B mode image. The display unit 34 displays the mechanical properties of a target bone to enable a user to evaluate the union or the strength of a fractured bone.

A pressing mechanism 50 is configured to apply a predetermined load to the shinbone 104 (i.e., a target bone to be diagnosed). As illustrated in Fig. 1, two probes 14 are disposed at both sides of a pressing position of the pressing mechanism 50. Each probe 14 can measure a displacement of the shinbone 104 caused when the shinbone 104 is pressed by the pressing mechanism 50.

The deflection amount, or another physical amount indicating mechanical properties, varies significantly depending on an applied load and a pressing position. For example, if the shinbone 104 (target bone) is in a fractured state, the strength of the bone 104 varies significantly depending on the distance from a fractured portion. Therefore, even if the applied load is set to the same value, the measured deflection varies significantly depending on the pressing position. If the deflection amount varies according to the pressing position, a user cannot accurately evaluate the strength of a target bone or the union of a fractured bone. Hence, the user determines a pressing position of the pressing mechanism for each measurement target considering the position of a fracture portion or the shape of a target bone. Then, to accurately perform the diagnosis of mechanical properties, the user is required to accurately press the determined pressing position.

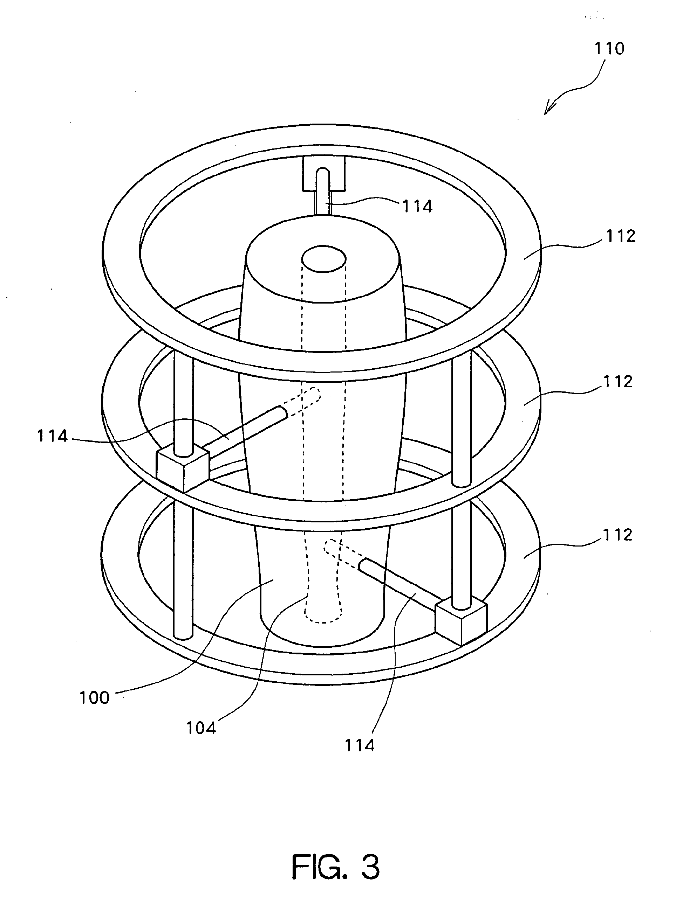

A user can evaluate the union of a fractured bone based on a diagnosis result of mechanical properties. In this case, the fractured bone may be fixed by an external fixation device. Fig. 3 illustrates a perspective view of an example external fixation device 110. The external fixation device 110 includes a plurality of pins 114 which are inserted into a human body to fix the fractured bone in position and supported by metal fittings 112. As will be apparent from Fig. 3, when each pin 114 fixes the fractured bone at one end, the other end of the pin 114 and the metal fittings 112 holding the pin 114 are positioned outside the body.

If the external fixation device 110 is attached around a target body, a conventional pressing mechanism interferes with the external fixation device 110 and cannot appropriately press a pressing position determined based on the position of a fracture portion and the shape of a target bone. Hence, the pressing mechanism 50 according to the present embodiment is configured to overcome the above-described problems.

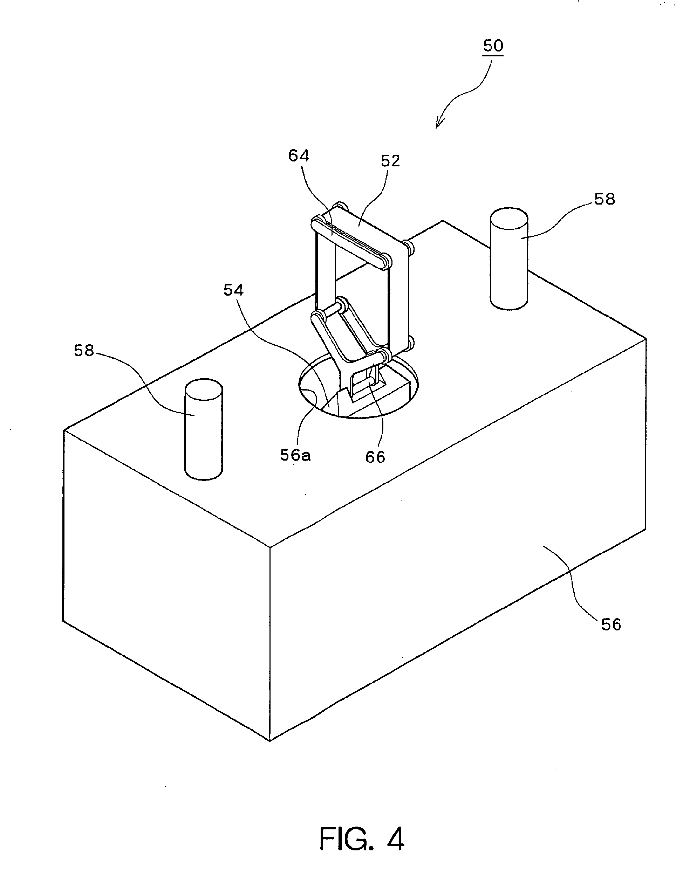

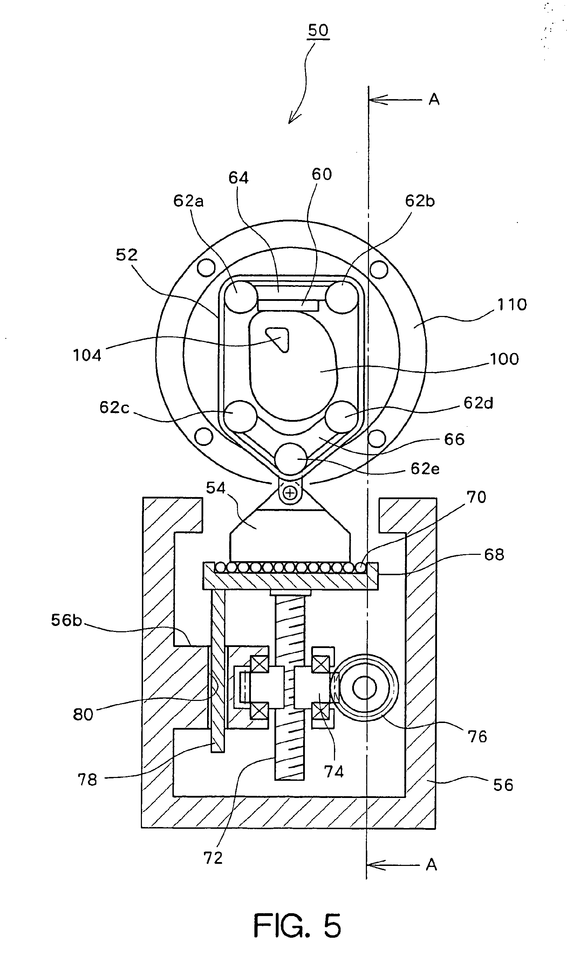

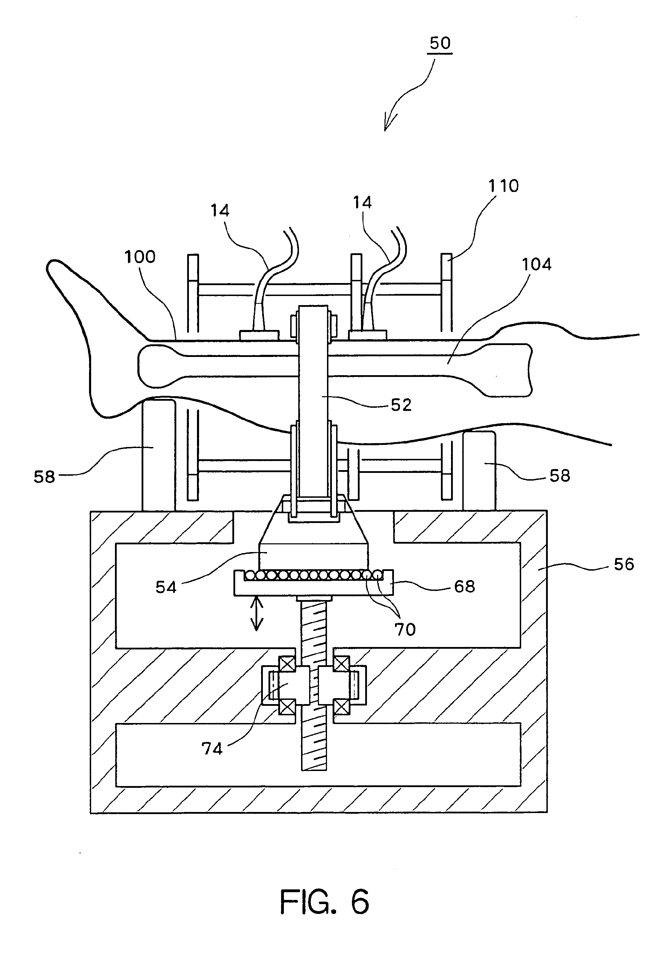

Fig. 4 illustrates a perspective view of the pressing mechanism 50 according to an embodiment. Fig. 5 illustrates a cross-sectional view of the pressing mechanism 50. Fig. 6 is a cross-sectional view taken along a line A-A of Fig. 5. The pressing mechanism 50 is configured to apply a pressing force to the shinbone 104 (target bone) by hanging a deadweight 54 having a predetermined weight value on the leg 100 which includes the shinbone 104. The deadweight 54 is connected to an annular belt 52 which is detachable from the leg 100. The annular belt 52 is a belt-like member having separable/connectable end parts equipped with an appropriate fastener (e.g., hook). The annular belt 52 becomes an endless shape when the end parts of the belt-like member are connected to each other by the fastener.

More specifically, before attaching the annular belt 52 around the leg 100, a user disengages the fastener of the annular belt 52. In this state, the belt 52 is in an extended state with separated end parts. Next, the user ties the belt 52 around the leg 100 loosely and engages the fastener to connect the belt 52 into an endless shape. In this manner, the annular belt 52 can be temporarily extended into a belt with separated end parts and can be easily attached around the leg 100 even in a state where the leg 100 is supported by the external fixation device 110.

A pressing board 60 is made of a rigid member and is fixedly bonded to an inner surface of the annular belt 52. The pressing board 60 (pressing member) can apply a pressing force to the shinbone 104 when it is brought into contact with a surface of the leg 100. Namely, a user adjusts the position of the annular belt 52 attached around the leg 100 so that the pressing board 60 is brought into contact with a predetermined pressing position. In this state, if the annular belt 52 is pulled downward by the deadweight 54, the pressing board 60 can apply a pressing force to the leg 100 (namely, the shinbone 104 included in the leg 100).

The annular belt 52 is entrained around five rollers 62 disposed in a pentagonal shape so that an appropriate tension acts on the annular belt 52. The five rollers 62 are appropriately spaced from an outer surface of the leg 100. The annular belt 52, when entrained around the rollers 62, does not directly contact the leg 100. In other words, the annular belt 52 can maintain good balance without causing any lack of uniformity due to frictional force.

Two rollers 62a and 62b are positioned at both sides of the pressing board 60 and are supported and connected by an upper metal holder 64 extending parallel to the pressing board 60. The upper metal holder 64 and the pressing board 60 are physically connected with each other. Thus, the positional relationship between upper metal holder 64 (namely, the rollers 62a and 62b connected to the upper metal holder 64) and the pressing board 60 can be regulated. Two rollers 62a and 62b are symmetrical with respect to the pressing position in the right-and-left direction. Furthermore, two rollers 62a and 62b are in a fixed positional relationship relative to the annular belt 52. In this respect, two rollers 62a and 62b function together as a "fixed roller group."

On the other hand, three rollers 62c, 62d, and 62e are supported and connected by a lower metal holder 66 configured into a Y shape. Two rollers 62c and 62d are symmetrical with respect to the roller 62e in the right-and-left direction. More specifically, three rollers 62c, 62d, and 62e are disposed in a reversed isosceles triangular shape. Three rollers 62c, 62d, and 62e can slide along an inner surface of the annular belt 52. If any unbalance occurs in the gravity acting on the annular belt 52, three rollers 62c, 62d, and 62e automatically move to a position where the gravity acting on the annular belt 52 is balanced. As a result, the downward force acting on the annular belt 52 can be automatically adjusted to be symmetrical in the right-and-left direction. Namely, three rollers 62, 62d, and 62e can slide along the inner surface of the annular belt 52 when the gravity is unbalanced in the right-and-left direction and adjust the gravity acting on the annular belt 52 to be symmetrical with respect to the pressing position. In this respect, three rollers 62, 62d, and 62e function as a "movable roller group."

The deadweight 54 having a predetermined weight is attached to a proximal end of the lower metal holder 66. When the deadweight 54 is pulled downward due to its gravity, a downward pressing force is applied to the annular belt 52 as well as to the pressing board 60 bonded together with the annular belt 52. The downward pressing force acts on the shinbone 104 (i.e., target bone). The weight of the deadweight 54 is determined considering a load value to be applied to a target bone. If the purpose of diagnosis or the type of a target bone is changed, the deadweight 54 can be replaced with another deadweight having a different weight value.

A base 68, movable in the up-and-down direction, is positioned under the deadweight 54. If no pressing force is required for the shinbone 104, the mobile base 68 can move upward to a higher position where the base 68 supports the bottom of the deadweight 54. In the raised state where the mobile base 68 mounts and supports the deadweight 54, no downward force acts on the annular belt 52 and the pressing board 60. On the other hand, if a pressing force acting on the shinbone 104 is required, the mobile base 68 can move downward to a lower position where the deadweight 54 separates from the mobile base 68 and is kept in a free state. In this state, the deadweight 54 pulls the annular belt 52 with the pressing board 60 downward due to its gravity. The pulling force (i.e. , pressing force) acts on the shinbone 104.

A plurality of steel balls 70, provided on an upper surface of the mobile base 68, enables the deadweight 54 to easily move in the horizontal direction relative to the mobile base 68. Namely, to accurately diagnose mechanical properties of the shinbone 104, it is desirable to subject the shinbone 104 to only the force acting in the vertical direction. In other words, it is desirable to eliminate any deviation in the direction of the pressing force acting on the shinbone 104. In a state where the deadweight 54 is mounted on the mobile base 68, the deadweight 54 does not constantly exist directly below the pressing board 60 and therefore the deadweight 54 may be offset from the pressing board 60.

If the mobile base 68 moves downward from the above-described state, the deadweight 54 is brought into a free state and the deadweight 54 automatically moves to a balanced position due to its gravity and stays directly below the pressing board 60. In this movement, the pressing board 60 is subjected to not only a vertical force but also a horizontal force. As a result, a significant deviation is caused in the direction of the force acting on the shinbone 104. To reduce such a deviation, the deadweight 54 is required to be constantly held at the position directly below the pressing board 60 in a state where the deadweight 54 is mounted on the mobile base 68. Hence, the present embodiment provides a plurality of steel balls 70 on the upper surface of the mobile base 68 so that the deadweight 54 can easily change the horizontal position on the mobile base 68 in accordance with the movement of the pressing board 60 and the annular belt 52.

The up-and-down movement of the mobile base 68 can be realized by a motor (i.e., a driving source not illustrated) and a transmission mechanism capable of transmitting driving force of the motor to the mobile base 68. The transmission mechanism includes a screw 72 extending downward from the bottom surface of the mobile base 68, a wheel 74 mating with the screw 72, and a worm wheel 76 mating with the wheel 74. The worm wheel 76 is connected to the output shaft of the motor and driven by the motor. The wheel 74 rotates when the worm wheel 76 rotates. The screw 72 rotates and shifts in the axial direction when the wheel 74 rotates. Thus, the mobile base 68 connected to the screw 72 can move in the up-and-down direction. The above-described arrangement of the driving mechanism is merely an example, and can be appropriately modified if necessary.

The mobile base 68 and the transmission mechanism are accommodated in a base body 56. The base body 56 is a hollow box member. The base body 56 includes an intermediate support 56b protruding in the horizontal direction from the inner wall. The intermediate support 56b includes a guide hole 80 extending in the vertical direction. A guide shaft 78, extending downward from the bottom surface of the mobile base 68, is inserted into the guide hole 80. The guide shaft 78 and the guide hole 80 cooperatively function as "guide unit" configured to regulate the moving direction of the mobile base 68.

An internal space of the base body 56 communicates with an external space via an opening 56a formed on an upper surface of the base body 56. The opening 56a has a sufficient size so that the deadweight 54 can be inserted via the opening 56a into the internal space of the base body 56. The upper surface of the base body 56 is covered by a steel or other ferromagnetic member. A mount base 58, including a magnet, is detachably mounted on the upper surface of the base body 56. The mount base 58 supports the leg 100 in which the shinbone 104 is present. Each mount base 58 has a sufficient height so that the external fixation device 110, when attached around the leg 100, does not interfere with the upper surface of the base body 56. However, the number of mount bases 58 and the shape of each mount base 58 can be appropriately changed considering the type of target bone or the shape of the external fixation device 110.

A user can perform the diagnosis of mechanical properties of the shinbone 104, using the above-described pressing mechanism 50 in the following manner. First, as a preparation for the diagnosis, a user determines a pressing position and a pressing force beforehand according to the purpose of the diagnosis and the type of bone (diagnosis object) and also considering the presence of any bone fracture. Then, the user selects the deadweight 54 having an appropriate weight value corresponding to the pressing force determined beforehand and hangs the selected deadweight 54 on the lower metal holder 66 of the pressing mechanism 50. Furthermore, the user raises the mobile base 68 to a higher position where the bottom of the deadweight 54 can be supported by the mobile base 68.

Then, the user puts the diagnosis object (the leg 100) on two mount bases 58 placed on the upper surface of the base body 56, so that the pressing position determined beforehand can be roughly positioned above the opening 56a.

Subsequently, the user ties the annular belt 52 around the leg 100. More specifically, before attaching the annular belt 52 around the leg 100, the user temporarily disengages the fastener of the annular belt 52. Next, the user ties the belt 52 (in an extended state with separated end parts) around the leg 100 loosely and engages the fastener to connect the belt 52 into an endless shape. At this moment, the deadweight 54 is supported by the mobile base 68. Therefore, no downward force acts on the annular belt 52 and the pressing board 60, and no pressing force is applied to the shinbone 104.

Then, the user performs a positional adjustment for the annular belt 52 so that the pressing board 60 can be accurately positioned at the pressing position determined beforehand. Furthermore, in addition to the positional adjustment for the annular belt 52, the user can adjust the position of the deadweight 54 connected to the annular belt 52 so that the deadweight 54 can be positioned directly below the pressing board 60 in the vertical direction. The plurality of steel balls 70 provided on the upper surface of the mobile base 68 can reduce frictional force caused when the deadweight 54 moves. Therefore, the user can easily perform the positional adjustment for the deadweight 54. In this case, instead of manually moving the deadweight 54, a user can temporarily lower the mobile base 68 to a position where the deadweight 54 is kept in a free state and can automatically move to a correct position.

After completing the positional adjustment for the pressing board 60 and the deadweight 54, the user attaches the probes 14 on an upper surface of the leg 100 at both sides of the pressing board 60 (i.e., pressing position).

Then, the user causes the ultrasound diagnostic apparatus 10 to start transmission/reception of ultrasonic waves using the probes 14. The ultrasound diagnostic apparatus 10 obtains surface shape data of the shinbone 104 in a state free from any pressing force, based on echo signals obtained by transmission/reception of ultrasonic waves.

Subsequently, the user moves the mobile base 68 downward while the ultrasound diagnostic apparatus 10 continues transmission/reception of ultrasonic waves. If the deadweight 54 is brought into a free state, the downward force (i.e., the gravity of the deadweight 54) acts on the annular belt 52 as well as on the pressing board 60 bonded to the annular belt 52. Thus, a downward pressing force acts on the shinbone 104.

If a pressing force is applied to the shinbone 104, the shinbone 104 causes a deformation in shape (e.g., deflection). The ultrasound diagnostic apparatus 10 obtains surface shape data of the shinbone 104 in a state where the shinbone 104 is subjected to the pressing force, based on echo signals obtained by transmission/reception of ultrasonic waves. After completing the measurement of surface shape data, the user moves the mobile base 68 upward to release the shinbone 104 from the gravity of the deadweight 54. Meanwhile, the ultrasound diagnostic apparatus 10 calculates a mechanical property value (e.g., deflection amount) of the shinbone 104 based on the surface shape data obtained before and after application of a pressing force. If the mechanical property value is obtained, the user completes the diagnosis of mechanical properties.

As will be apparent from the foregoing description and Figs. 4 through 6, in the pressing mechanism 50 according to the present embodiment, the components located in the vicinity of a pressing position are limited to the annular belt 52, the pressing board 60, and the roller 62, which are compact in size. Therefore, even if the pins 114 and the metal fittings 112 (i.e., constituent members of the external fixation device 110) are present in the vicinity of the pressing position, the pressing mechanism 50 can accurately press a designated pressing position without causing any interference with the constituent members of the external fixation device 110. As a result, the present embodiment enables a user to appropriately perform the diagnosis of mechanical properties even in a state where the external fixation device 110 is attached around a target body.

The arrangement of the above-described pressing mechanism is merely an example, and can be appropriately modified if an appropriate pressing force can be applied to a target bone when a deadweight having a predetermined weight value is hung. For example, a pressing mechanism according to another embodiment may not include the rollers 62, the pressing board 60, and the steel balls 70.

In the above-described embodiment, the mobile base 68 directly supports the deadweight 54. However, if an appropriate urging member is provided between the deadweight 54 and the mobile base 68, the urging member can gradually apply a force to the deadweight 54 in the upper direction. The pressing force can be slowly applied to the shinbone 104 via the urging member. The deflection amount of a bone or other mechanical property value is variable depending on how a pressing force is applied. Namely, a mechanical property value obtained when the pressing force is slowly applied is different from a mechanical property value obtained when the pressing force is quickly applied.

Furthermore, according to the purpose of mechanical diagnosis, it may be necessary to obtain a mechanical property value under a static load. In such a case, it is preferable to provide an urging member between the deadweight 54 and the mobile base 68 so that the pressing force can be slowly applied to the deadweight 54.

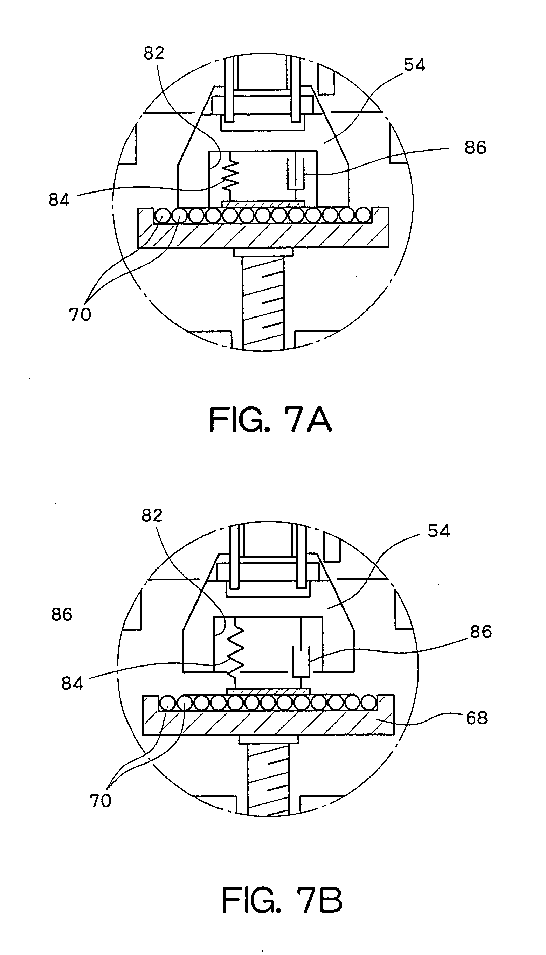

Figs. 7A and 7B illustrate a shock absorbing mechanism including a coil spring 84 and a damper 86 which are provided in a recessed portion 82 formed on the bottom of the deadweight 54. If a user slowly moves the mobile base 68 downward and the mobile base 68 starts separating from the deadweight 54, the shock absorbing mechanism can continue supporting the deadweight 54. The weight of deadweight 54 supported by the shock absorbing mechanism gradually decreases with increasing distance between the mobile base 68 and the deadweight 54. Finally, the entire weight of the deadweight 54 acts on the pressing board 60. Namely, if an appropriate shock absorbing mechanism is provided between the deadweight 54 and the mobile base 68, the load amount acting on the shinbone 104 can be gradually increased.

Furthermore, the above-described embodiment uses transmission/reception of ultrasonic waves to detect a change in the shape of a target bone subjected to a pressing force. However, according to another embodiment, indirectly measuring a change in the shape of a target bone is feasible. For example, a laser displacement gauge can be used to measure a bending amount of a body part in which a target bone is present (e.g., a leg if the target bone is a shinbone) and diagnose mechanical properties of the target bone based on a measurement result. In this case, although the tissues of a human body have some effect on a measurement result, a user can easily diagnose mechanical properties of a target bone.

Although the preferred embodiments of the present invention have been described using specific terms, such description is for illustrative purposes only, and it is to be understood that modifications and variations may be made without departing from the spirit or scope of the appended claims.

## Claims

1. A pressing mechanism configured to apply a pressing force to a target bone when a diagnosis of mechanical properties is performed for the target bone, comprising:
an annular belt detachably hung around a diagnosis object in which the target bone is present;
a deadweight connected to a lower end of the annular belt and having a weight value corresponding to a desired pressing force; and
a mobile base positioned under the deadweight and movable in an up-and-down direction,
wherein if the mobile base moves downward the deadweight separates from the mobile base and is brought into a free state where a pressing force acts on the target bone, and if the mobile base moves upward the deadweight is supported by the mobile base and the target bone is released from the pressing force.

2. The pressing mechanism according to claim 1, wherein the annular belt is entrained around a plurality of rollers which are spaced from the diagnosis object.

3. The pressing mechanism according to claim 2, wherein the rollers include a group of fixed rollers which are symmetrically disposed with respect to a pressing position in the right-and-left direction and are in a fixed relationship relative to the annular belt, and a group of movable rollers which can slide along an inner surface of the annular belt and automatically move to a position where the gravity acting on the annular belt is balanced in the right-and-left direction.

4. The pressing mechanism according to claim 1, wherein the mobile base supports the deadweight via a plurality of balls that permits horizontal movement of the deadweight relative to the mobile base.

5. The pressing mechanism according to claim 1, further comprising an urging member disposed between the mobile base and the deadweight, to urge the deadweight in the upper direction.

6. An ultrasound diagnostic apparatus configured to transmit and receive ultrasonic waves to and from a target bone in a state where a pressing force is applied to the target bone, and obtain deformation properties of the target bone subjected to the pressing force, comprising:
a pressing mechanism configured to press a predetermined position on a target bone;
an ultrasonic probe configured to transmit and receive ultrasonic waves to and from the target bone; and
a calculation unit configured to calculate a change in the target bone before and after the target bone is pressed by the pressing mechanism based on an echo signal obtained via the ultrasonic probe that performs transmission and reception of ultrasonic waves,
wherein the pressing mechanism comprises:
an annular belt detachably hung around a diagnosis object in which the target bone is present;
a deadweight connected to a lower end of the annular belt and having a weight value corresponding to a desired pressing force; and
a mobile base positioned under the deadweight and movable in an up-and-down direction, wherein if the mobile base moves downward the deadweight separates from the mobile base and is brought into a free state where a pressing force acts on the target bone, and if the mobile base moves upward the deadweight is supported by the mobile base and the target bone is released from the pressing force.
